# EUROPEAN PATENT APPLICATION

(11) **EP 2 301 465 A1**
(43) Date of publication of application: **30.03.2011**
(21) Application number: 10182003.3
(22) Date of filing: 24.01.2007
(51) Int. Cl.: A61B 18/12

(54) **Method for transmitting data across patient isolation barrier**

(30) Priority: 24.01.2006 US 338479
(62) Divisional of application: 07001489.9
(71) Applicant: Covidien AG, 8212 Neuhausen am Rheinfall (CH)
(72) Inventor: Keppel, David S., Longmont, CO 80501 (US)
(74) Representative: HOFFMANN EITLE

(57) **Abstract**

Systems and methods for controlling an electrosurgical generator through a patient isolation barrier are disclosed. The system includes a control assembly disposed within a surgical hand piece configured to control the generator. The control assembly has one or more input devices and one ore more resistor assemblies in electrical communication with a DC voltage source which supplies a control current. The input devices are configured to adjust resistance of the resistor assemblies and voltage of the control current passing therethrough. The system also includes an isolation barrier transmitter having a voltage-to-frequency converter which converts the voltage passing through the resistor assembly into a corresponding frequency. The transmitter transmits the frequency across the patient isolation barrier. The system further includes an isolation barrier receiver configured to receive the frequency and to convert the frequency into a control signal and a microprocessor which processes the control signal and controls the generator based on the control signal.

## Description

### BACKGROUND

### 1. Technical Field

The present disclosure is directed to electrosurgical systems, and, more particularly, to a method and system for transmitting data across isolation barrier, wherein the data is used for controlling electrosurgical generators.

### 2. Background of Related Art

Electrosurgical generators are employed by surgeons in conjunction with electrosurgical instruments to perform a variety of surgical procedures including tissue division. An electrosurgical generator generates and modulates electrosurgical energy which is applied to the tissue by an electrosurgical instrument. Generators operate in a plurality of modes, e.g., cut, coagulation, or blend. These modes are accomplished by using different current waveforms. Using a constant waveform, the generator allows a surgeon to vaporize or cut tissue since a constant waveform produces heat very rapidly. Using an intermittent waveform causes the generator's duty cycle to be reduced to coagulate tissue. A blended current allows for a mixture of the two above-mentioned waveforms to achieve intermediate results.

Conventionally, the capability of changing operating modes and output power has been provided by controls located at a keyboard or control panel of the generator. However, this method is very inconvenient for the surgeon, since to make any modifications to the operating current, the surgeon has to return to the control panel of the generator, which is outside the sterile field. This requires the surgeon to divert attention away from the operative site. To provide for better control mechanisms which could be used within the sterile field, electrosurgical systems have later included a hand piece designed to be held by the surgeon with controls disposed thereon. The hand piece allowed the surgeon to change the mode of operation and the power output of the generator without leaving the sterile field and without diverting their attention from the operative site.

Conventional hand pieces included a plurality of switches and transmitted control signals corresponding to switch positions across an isolation patient barrier. The barrier separated circuitry connected to the patient from the circuitry connected to the generator to prevent accidental electrical shocks to the patient. Control signals crossed this barrier by a variety of means, including magnetic coupling, optical coupling, and capacitive coupling. However, current hand pieces utilize resistive element networks to provide for greater range of control selection with fewer wires, instead of relying on switch activation signals which required corresponding wiring.

Therefore there is a need for a method and system to transmit analog voltage produced by resistive element networks across a patient isolation barrier without using expensive analog components while preserving the safety barrier.

### SUMMARY

Disclosed are systems and methods for controlling an electrosurgical generator through a patient isolation barrier. The system includes a hand piece in electrical communication with the generator. The hand piece includes controls for controlling and/or adjusting operational functions of the generator, including the operating mode and the intensity of the output energy. The controls include one or more input device(s) coupled to a resistor assembly which is connected to a DC voltage source to supply control current. Activation of the input device adjusts the resistance of the resistor assembly thereby adjusting the voltage. The voltage is converted into a corresponding frequency by a voltage-to-frequency converter and is then transmitted across the patient isolation barrier through an optical coupler. The frequency is either converted directly into a control signal or back into voltage which is then converted into the control signal. The control signal is received by a microprocessor, which then adjusts the generator based on the control signal.

According to one embodiment of the present disclosure, a system for controlling an electrosurgical generator through a patient isolation barrier is disclosed. The system includes a control assembly disposed within a surgical hand piece configured to control the generator. The control assembly has one or more input devices and one ore more resistor assemblies in electrical communication with a DC voltage source which supplies a control current. The input devices are configured to adjust resistance of the resistor assemblies and voltage of the control current passing therethrough. The system also includes an isolation barrier transmitter having a voltage-to-frequency converter which converts the voltage passing through the resistor assembly into a corresponding frequency. The transmitter transmits the frequency across the patient isolation barrier. The system further includes an isolation barrier receiver configured to receive the frequency and to convert the frequency into a control signal and a microprocessor which processes the control signal and controls the generator based on the control signal.

Another aspect of the present disclosure relates to a method for controlling an electrosurgical generator through a patient isolation barrier. The method includes the step of activating one or more input devices disposed within a surgical hand piece configured to control the generator. The hand piece includes one or more resistor assemblies in electrical communication with a DC voltage source which supplies a control current. The input devices are configured to adjust resistance of the one or more resistor assemblies and voltage of the control current passing therethrough. The method also includes the steps of converting the voltage into a corresponding frequency through a voltage-to-frequency converter, transmitting the frequency across the patient isolation barrier transmitter. The method further includes the steps of converting the frequency into a control signal and transmitting the control signal to a microprocessor which is configured to control the generator based on the control signal.

A further aspect of the present disclosure relates to an electrosurgical instrument. The instrument includes a control assembly disposed within a surgical hand piece configured to control an electrosurgical generator. The control assembly has one or more input devices and one ore more resistor assemblies in electrical communication with a DC voltage source which supplies a control current. The input devices are configured to adjust resistance of the resistor assemblies and voltage of the control current passing therethrough. The instrument also includes an isolation barrier transmitter having a voltage-to-frequency converter which converts the voltage passing through the resistor assembly into a corresponding frequency. The transmitter transmits the frequency across the patient isolation barrier. The instrument further includes an isolation barrier receiver configured to receive the frequency and to convert the frequency into a control signal readable by a microprocessor.

### BRIEF DESCRIPTION OF THE DRAWINGS

Various embodiments will be described herein below with reference to the drawings wherein:
Fig. 1 is a block diagram illustrating in general an electrosurgical system;
Fig. 2 is a block diagram of a hand piece and an electrosurgical generator in accordance with the present disclosure;
Fig. 3 is a schematic diagram of illustrative circuitry in accordance with the present disclosure; and
Fig. 4 is a flow diagram of a method for transmitting control data across a patient isolation barrier.

### DETAILED DESCRIPTION

Reference should be made to the drawings where like reference numerals refer to similar elements throughout the various figures. Embodiments of the present disclosure are described herein below with reference to the accompanying drawings. In the following description, well-known functions or constructions are not described in detail to avoid obscuring the present disclosure in unnecessary detail.

The present disclosure provides a system and method for controlling an electrosurgical generator through a hand piece, and more specifically, for passing input signals across a patient isolation barrier, where the input signals are in the form of analog and/or variable voltage. The hand piece includes an input device connected to a resistor assembly (e.g., a resistor network) which is in electrical communication with a DC voltage source. The DC voltage source supplies control current which upon passing through the resistor assembly is modified. The resistance of the resistor assembly varies depending on the inputs of the input device. The resistance modifies the voltage of the control current which thereafter is converted to a corresponding frequency by a voltage-to-frequency converter. The frequency is then transmitted across the patient isolation barrier through an optical coupler. The frequency is then either converted into a digital control signal by an analog-to-digital converter or back into voltage which is then subsequently converted into the digital control signal. The digital control signal is then relayed to a microprocessor which controls the generator based on the control signal.

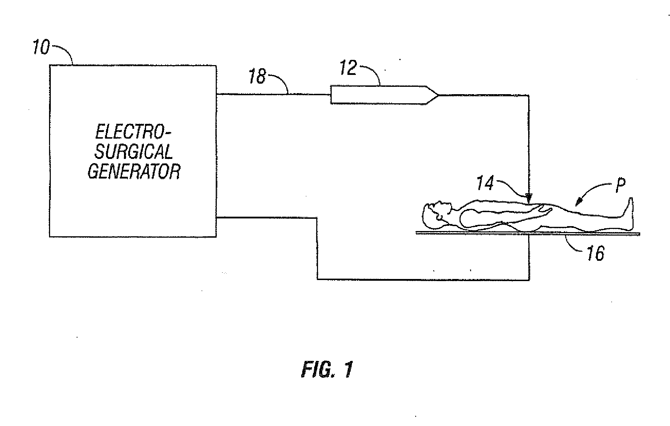

Fig. 1 shows an electrosurgical system including a generator 10 and a hand piece 12 having an active electrode 14 which is brought in contact with a patient P to effect a cut and/or coagulation procedure depending upon the operating mode selected. Attached to the patent P is a return electrode 16 which returns the electrosurgical current from the patient P to the generator 10.

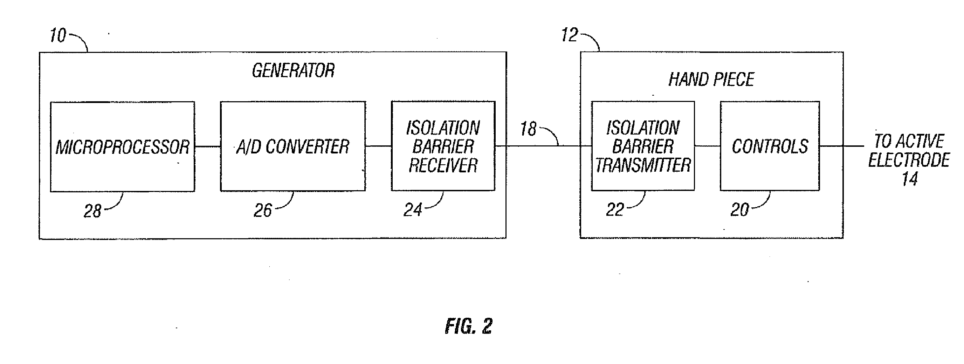

Referring to Fig. 2, the generator 10 and the hand piece 12 are shown in more detail. The generator 10 also includes a keyboard and/or control panel (not shown) for adjusting functions thereof. The hand piece 12 is connected to the generator 10 by a cable 18 which includes a plurality of wires for transmitting electrical energy. One of the wires conducts electrosurgical current from the generator 10 to the active electrode 14, hereafter referred to as the active line. The active line also conducts DC voltage from the generator 10 which is employed by the hand piece 12 to transmit input signals back thereto. A DC voltage source may be used to transmit a control current which is used by the hand piece 12 to transmit input signals to the generator 10. Using DC voltage to transmit input signals is well known in the art and is described in commonly owned U.S, Patents Nos. 3,699,967 and 3,801,800, both of which are hereby incorporated by reference in their entirety herein.

The hand piece 12 includes a control assembly 20 having a plurality of input devices (e.g., buttons, switches, etc.) for adjusting intensity of the electrosurgical energy and selecting the operating mode (e.g., cut, coagulation, blend). The control assembly 20 provides complementary and/or redundant controls for the generator 10 which may be used to adjust the intensity of the energy output by the generator, change the operating mode (e.g., cut, coagulate, blend). The control assembly 20 includes a plurality of resistive elements coupled to the inputs so that the inputs correspond to specific resistances. This allows for fewer wires to be used within the control assembly 20 since different input signals (e.g., corresponding voltage) can be transmitted along a single wire by varying the resistance thereby varying the voltage of the control current.

The hand piece 12 also includes an isolation barrier transmitter 22 which creates a physical isolation barrier between the hand piece 12 and the generator 10. The transmitter 22 receives the voltage signals from the control assembly 20 and converts the variable voltage (e.g., based on variable resistance) into a corresponding frequency. The frequency is then communicated across a patient isolation barrier optically, magnetically or capacitively and transmitted to the generator 10 along one of the wires within the cable 18. The patient isolation barrier refers to a physical gap in the electrical connections between the control assembly 20 of the hand piece 12 and the generator 10 to prevent accidental electrical shock to the patient. Transmittal of control signals across the physical gap may, therefore, be accomplished by using optical couplers and or magnetic couplers which are within the purview of those skilled in the art.

Those skilled in the art will appreciate that the transmitter 22 may be disposed within the generator 10 or another device, since the patient isolation barrier would still exist, although at a different location. The control assembly 20 may be disposed within the hand piece 12 so that the surgeon has convenient access to input mechanisms for controlling the generator 10.

The generator 10 includes an isolation barrier receiver 24 which receives the frequency corresponding to the input voltage from the control 20 and converted by the transmitter 22. The receiver 24 transmits the frequency signal to an analog-to-digital (A/D) converter 26, which then converts the analog frequency signal into a control signal and transmits the digital control signals to a microprocessor 28. The microprocessor 28 is responsible for controlling functions of the generator 10 (e.g., adjusting energy output, receiving control signals, analyzing sensor outputs, etc.). More specifically, the microprocessor 28, upon receiving digital input signals makes corresponding adjustments within the generator 10.

It is envisioned that the receiver 24 may also convert the frequency into voltage using a frequency-to-voltage converter and then transmit voltage signal to the analog-to-digital (A/D) converter 26, which then transmits the control signal to a microprocessor 28.

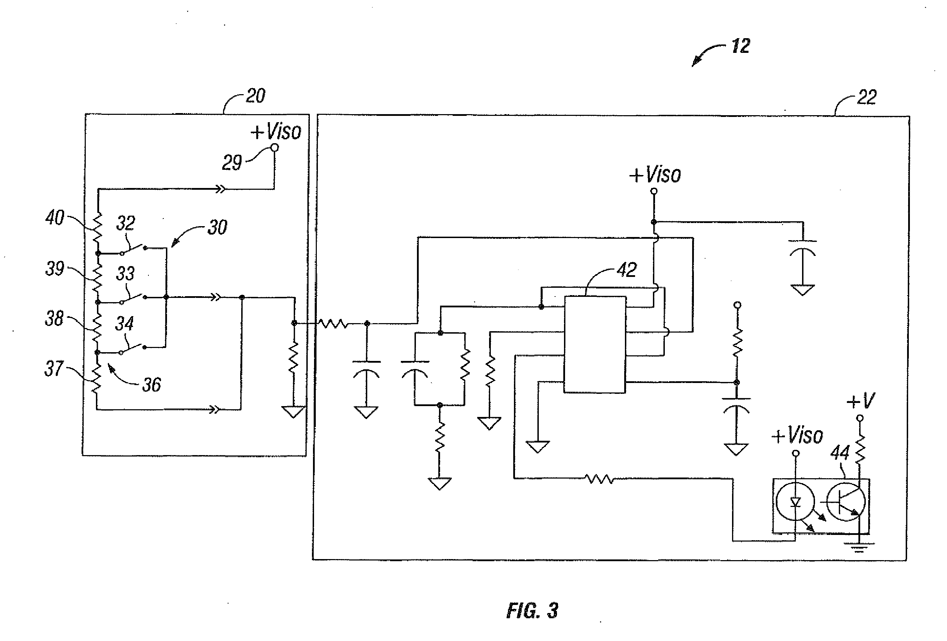

Fig. 3 shows an electrical schematic of the hand piece 12 having the control assembly 20 and the transmitter 22. The control assembly 20 provides input for one of a plurality of input categories, such as mode selection, intensity, etc. It is envisioned that each of the input categories would include corresponding controls and isolation barrier transmitters. Those skilled in the art will appreciate that the number of controls and transmitters may correspond to the amount of input categories (e.g., one set of controls and transmitters for intensity controls, another set of controls for operating mode selectors, etc.). However, it is also possible to include only one instance of controls and a corresponding transmitter to provide signals for a plurality of input categories. In that instance, it is envisioned that the A/D converter 26 is also configured to differentiate among a wide range of resistances.

As discussed above, the input signals are communicated by using variable resistance. A DC power supply (not shown) communicates DC voltage +Viso through a connection 29 to the control assembly 20. The control assembly 20 includes a switch assembly 30 which includes a plurality of switches (e.g., switches 32, 33, 34). The control assembly 20 may include input mechanisms for controlling any of the properties of the generator 10. For instance, the control assembly 20 may adjust the intensity of the energy supplied by the generator 10. The intensity may be controlled by adjusting the intensity by a specified percentage (e.g., 10%). It should be noted, in accordance with the present disclosure, the increase in power is not a ten percent increase in the current power but rather the increase is ten percent of the power last set at the keyboard or control panel of the generator 10. If the power were increased by ten percent of the current power, certain difficulties could arise. For example, if the initial power setting is 100 watts and the increments are ten percent of the current output power, the first increase would increment the output power to 110 watts. The next increment would increase it to 121 watts. If the power were now decremented by ten percent, it would be decreased to about 109 watts and a further decrement would decrease it to about 98 watts. Hence, it can be seen that, although the initial power was 100 watts, the power, after two increases of ten percent each and two decreases of ten percent, is 98 watts. The operator would expect the power to return to the initial power after such a sequence and not to jump to a different value.

To avoid this difficulty, the procedure of the present disclosure is employed where the last output power set at the control panel of the generator is employed as a reference point for subsequent changes in power. Although these changes have been described in terms of ten percent increments or decrements, the percentage change may be, of course, other than ten percent. Further, the percentage change may vary from step to step if so desired.

In summary, if the initial power setting from the keyboard or control panel of the generator is 100 watts and the surgeon increases that power from the hand switch to 150 watts by five ten percent increments, no further increases from the hand piece 12 are possible and the surgeon must return to the keyboard to change the power setting thereat. Assume the surgeon changes the setting to 200 watts at the control panel. If he then subsequently increments the power by a ten percent factor, the power increase will be 20 watts since the last output power set at the control panel was 200 watts. It will not be 10 watts which is ten percent of the original output power setting at the control panel.

Referring back to Fig. 3 and in reference to the above example where the control assembly 20 adjusts the intensity of the energy, the closing of the switch 32 corresponds to an input configured to increase the intensity by 10%, the closing of the switch 33 corresponds to a decrease the intensity by 10%, and the closing of the switch 34 corresponds to energy shut off. Depending on which of the switches 32, 33, 34 are closed the voltage passing through the resistor assembly 36 having resistors 37, 38, 39, 40 will vary.

More specifically, closing of the switch 32 results in a resistance R₁, Similarly, closing of the switch 33 results in a resistance R₂ and closing of the switch 34 results in resistance R₃. By varying the resistance within the resistor assembly 36, the control assembly 20 modifies the DC voltage passing through the resistor assembly 36. More simply put, each of the inputs at the control assembly 20 closes one of the corresponding switches 32, 33, 34 thereby setting one of the resistances R₁, R₂, R₃ at the resistor assembly 36 and modifying the DC voltage passing therethrough so that the voltage changes accordingly. Thus, each of the inputs has a corresponding voltage (e.g., V₁, V₂, V₃).

The corresponding voltage passes to the transmitter 22 which converts the voltage into frequency and transmits the frequency across the physical isolation barrier. The transmitter 22 includes a voltage-to-frequency converter (VFC) 42 connected to an optical coupler 44. An exemplary voltage-to-frequency converter may be the Precision VFC model no. LM331, manufactured by National Semiconductor Corp. located at 2900 Semiconductor Drive, P.O. Box 58090, Santa Clara, California. The circuitry schematic of the VFC model No. LM331 and the operation thereof is provided in its product manual which is hereby incorporated by reference herein in its entirety. The manual can be found on the National Semiconductor Corp's website at cache.national.com/ds/LM/LM231.pdf.

The VFC 42 converts the input voltage corresponding to the inputs at the control assembly 20 to a proportional frequency (e.g., f₁, f₂, f₃). The frequency is then transmitted to the optical coupler 44 which transfers the frequency signals from the transmitter 22 to the receiver 24 across the physical isolation barrier. The receiver 24 then relays the control frequency signal to the A/D converter 26 which converts the frequency signal into a digital control signal. The microprocessor 28 receives the corresponding control signal and analyzes it to determine a corresponding input command. For instance, frequency f₁ corresponds with increasing the intensity by 10% and adjusts the energy output of the generator 10 accordingly.

It is envisioned that the receiver 24 may include a frequency-to-voltage converter (not shown) to convert the frequency signal back into voltage. The receiver 24 then transmits the voltage signal to the A/D converter 26 which converts the frequency signal into a control signal. The microprocessor 28 thereafter issues a control command to the generator 10.

It is also envisioned that the switches 32, 33, 34 can be pressed continuously (e.g., pressing the switch 32 increases the intensity by 10% every half-second that the switch 32 is pressed). This does not deviate from the spirit of the disclosure since the corresponding voltage V₁ is still maintained and is later converted into the corresponding control signal.

It is further envisioned that the A/D converter 26 and the VFC 42 can be substituted by a frequency counting circuit (not shown) which measures the corresponding frequency and transmits a corresponding signal to the microprocessor 28 across the optical coupler 44 for determining the associated input command.

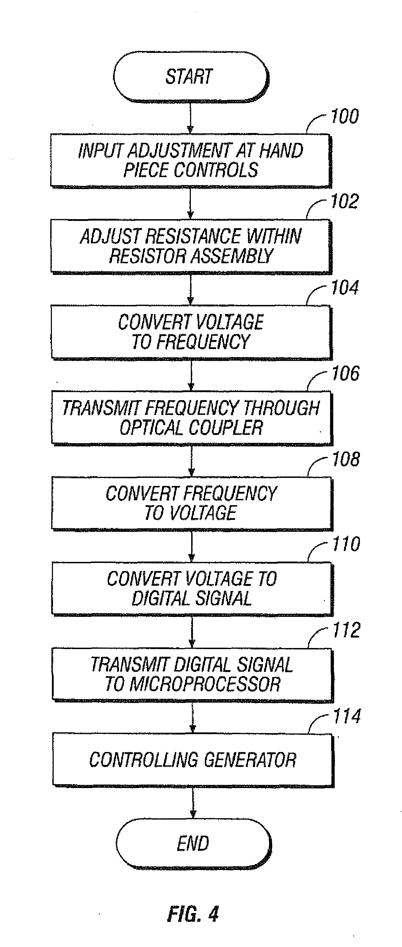

Fig. 4 is a flow diagram of a method for transmitting control data across a patient isolation barrier. In step 100, the surgeon inputs adjustments into the control assembly 20 (e.g., increasing the intensity by 10%) by pressing corresponding buttons and/or activating switches. In step 102, the pressing of buttons activates corresponding switches (e.g., one or more switches 32, 33, 34) which in turn modifies the resistance within the resistor assembly 36. The resistance adjusts the DC voltage passing through the resistor assembly 36 which corresponds to inputs at the control assembly 20.

In step 104, the modified DC voltage is converted by the VFC 42 into frequency and in step 106 the frequency is transmitted across the patient isolation barrier to the receiver 24. In optional step 108, the receiver 24 converts the frequency back into voltage using a frequency-to-voltage converter. The D/A converter, in step 110, converts the frequency or the voltage signals (if frequency was converted back into voltage) into a control signal and transmits the control signal to the microprocessor 28 in step 112. The microprocessor 28 matches the control signal with a corresponding input command and adjusts the generator 10 accordingly in step 114. For instance, in the above example, the surgeon increased the intensity by 10% therefore the microprocessor 28 would adjust the intensity correspondingly.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope and spirit of the claims appended hereto.

The following numbered items correspond to the claims as originally filed for the parent application (EP 1 810 631(A2)). They are included to preserve the original disclosure of the parent application in the present application.
1. A system tor controlling an electrosurgical generator through a patient isolation barrier, the system comprising:
   a control assembly disposed within a surgical hand piece configured to control the generator, the control assembly having at least one input device and at least one resistor assembly in electrical communication with a DC voltage source which supplies a control current, the at least one input device configured to adjust resistance of the at least one resistor assembly and voltage of the control current passing therethrough;
   an isolation barrier transmitter having a voltage-to-frequency converter which converts the voltage passing through the resistor assembly into a corresponding frequency, the transmitter transmits the frequency across the patient isolation barrier;
   an isolation barrier receiver configured to receive the frequency and to convert the frequency into a control signal; and
   a microprocessor which processes the control signal and controls the generator based on the control signals.
2. A system as in 1, further comprising:
   an analog-to-digital converter configured to convert the frequency into a digital control signal.
3. A system as in 1, wherein the isolation barrier transmitter includes a optical coupler which transmits the frequency across the patient isolation barrier.
4. A system as in 1, wherein the isolation barrier transmitter includes a capacitor which transmits the frequency across the patient isolation barrier.
5. A system as in 1, wherein the isolation barrier transmitter includes a magnetic coupling device which transmits the frequency across the patient isolation barrier.
6. A system as in 1, wherein the input device adjusts an operational mode of the generator.
7. A system as in 1, wherein the input device adjusts an output intensity of the generator.
8. A system as in 1, further comprising:
   a frequency-to-voltage converter which converts the frequency into the voltage, wherein the isolation barrier is further configured to convert the voltage into a control signal; and wherein the analog-to-digital converter is configured to convert the voltage into a digital control signal.
9. An electrosurgical instrument comprising:
   a control assembly disposed within a surgical hand piece configured to control an electrosurgical generator, the control assembly having at least one input device and at least one resistor assembly in electrical communication with a DC voltage source which supplies a control current, the at least one input device configured to adjust resistance of the at least one resistor assembly and voltage of the control current passing therethrough;
   an isolation barrier transmitter having a voltage-to-frequency converter which converts the voltage passing through the resistor assembly into a corresponding frequency, the transmitter transmits the frequency across the patient isolation barrier; and
   an isolation barrier receiver configured to receive the frequency and to convert the frequency into a control signal readable by a microprocessor.

## Claims

1. A method for controlling an electrosurgical generator through an isolation barrier, comprising the steps of:
activating at least one input device disposed within a hand piece to provide a user input signal configured to control the generator, the hand piece having at least one corresponding resistor assembly in electrical communication with a DC voltage source which supplies a control current, the at least one input device configured to adjust resistance of the at least one resistor assembly to adjust the voltage of the user input signal;
converting the voltage of the user input signal into a corresponding user input signal frequency through a voltage-to-frequency converter;
transmitting the user input signal frequency across the isolation barrier transmitter;
converting the user input signal frequency into a user input control signal; and
transmitting the user input control signal to a microprocessor which is configured to control the generator based on the user input control signal.

2. A method as in claim 1, further comprising the step of:
converting the user input signal frequency into a user input digital control signal through an analog-to-digital converter.

3. A method as in claim 1, wherein the isolation barrier transmitter includes an optical coupler which transmits the user input signal frequency across the isolation barrier.

4. A method as in claim 1, wherein the isolation barrier transmitter includes a capacitor which transmits the user input signal frequency across the isolation barrier.

5. A method as in claim 1, wherein the isolation barrier transmitter includes a magnetic device which transmits the user input signal frequency across the isolation barrier.

6. A method as in claim 1, wherein the input device adjusts a mode of the generator.

7. A method as in claim 1, wherein the input device adjusts an intensity of the generator.

8. A method as in claim 2, further comprising the steps of:
receiving and converting the user input signal frequency into a user input signal voltage through a frequency-to-voltage converter; and
converting the user input signal voltage into a digital control signal through the analog-to-digital converter.
